Europäisches Patentamt

**European Patent Office**

Office européen des brevets

(11) Publication number: **0 345 792**
**A2**

# EUROPEAN PATENT APPLICATION

(21) Application number: **89110414.3**

(22) Date of filing: **08.06.89**

(51) Int. Cl.⁴: **C12N 15/00 , C07K 15/04 ,**
**C12P 21/02 , G01N 33/569**

(30) Priority: **10.06.88 US 205401**

(43) Date of publication of application:
**13.12.89 Bulletin 89/50**

(84) Designated Contracting States:
**AT BE CH DE ES FR GB IT LI NL SE**

(71) Applicant: **F. HOFFMANN-LA ROCHE AG**
**Postfach 3255**
**CH-4002 Basel(CH)**

(72) Inventor: **Longiaru, Mathew**
**5 Weber Road**
**West Orange, N.J. 07052(US)**
Inventor: **Scherer, Bradley John**
**273 Upper Mountain Avenue**
**Upper Montclair, N.J. 07043(US)**
Inventor: **Terry, Robert William**
**R.D. 4 Box 55**
**Boonton, N.J. 07005(US)**

(74) Representative: **Lederer, Franz, Dr. et al**
**Patentanwalt Dr. Franz Lederer**
**Lucile-Grahn-Strasse 22**
**D-8000 München 80(DE)**

(54) HTLV-I / HIV-1 fusion proteins.

(57) Hybrid genes comprising a first DNA sequence coding for a polypeptide containing at least one epitope of the HIV-1 envelope protein and a second DNA sequence coding for a polypeptide containing at least one epitope of the HTLV-I envelope protein, the corresponding hybrid proteins, recombinant vectors encoding said hybrid proteins, unicellular hosts containing said recombinant vectors which are capable of producing said proteins, methods of producing said hybrid proteins using the unicellular hosts, and methods for detecting antibodies to HIV-1 and HTLV-I and HTLV-II in mammalian body fluids including a test kit for such purpose are disclosed.

figure 1

## HTLV-I / HIV-1 fusion proteins

The present invention relates to a hybrid gene containing a portion of the HIV-1 envelope gene and a portion of the HTLV-I envelope gene. The invention also relates to the corresponding gene products, the recombinant vectors encoding said gene products, unicellular hosts containing these recombinant vectors, methods for producing said gene products by using unicellular hosts containing the the recombinant vectors, and methods for detecting the presence of antibodies to HIV-1 and HTLV-I and HTLV-II envelope gene products in mammalian body fluids.

## BACKGROUND OF THE INVENTION

Human T Cell Lymphotropic Virus Type 1 (HTLV-I) is a Type C RNA virus which has been identified as the cause of Adult T-Cell Leukemia/Lymphoma (ATL). HTLV-I was the first retrovirus to be isolated and cloned from patients with ATL, and has also been linked to neurological diseases such as tropical spastic paraparesis and HTLV-I associated myelopathy. In certain areas of the world HTLV-I infections appear to be endemic. For example, in certain Southern Japanese populations the seroprevalence of HTLV-I is known to be as high as 35%. While the frequency of HTLV-I infection in the U.S. is still quite low, infection by HTLV-I alone or in combination with other human retroviruses appears to be a growing problem in the inner-city populations where intravenous drug abuse is a common practice. Another isolate, designated HTLV-II, derived from a patient with T-cell hairy cell Leukemia is closely related to HTLV-I.

Once the complete nucleotide sequence of the HTLV-I genome was determined, manufacture of the corresponding proteins followed, further enabling the detection of antibodies to these proteins. When techniques such as Western Blot and radioimmune precipitation (RIP) were adapted to measure human antibodies to HTLV-1 core and elvelope proteins, it became evident that HTLV-1 infection was nearly endemic in certain populations. It was also found that antibodies from a patient with hairy cell leukemia, whose disease was associated with HTLV-II, strongly reacted to a polypeptide corresponding with the p21 region of the HTLV-I envelope indicating the high degree of homology between HTLV-I and HTLV-II.

However, the importance of HTLV-I as a human pathogen has not been recognized because the AIDS epidemic has consumed the interest of researchers and clinicians. AIDS is due to infection by the HIV-1 retrovirus. Both HIV-1 and HTLV-I are transmitted in a variety of ways, which include contaminated needles, blood transfusions, sexual contact and vertical transmission from mother to offspring. Since both viruses are associated with human disease, investigators have now begun to screen cross-sections of the American population for antibodies to HTLV-I as well as HIV-1.

At present there are separate screening tests for antibodies to HTLV-I and HIV-1, both of which use disrupted virions obtained from virally transformed human T cell lines. The viral antigens are semipurified and coated onto a solid phase which facilitates binding of human antibodies, if present, to the viral antigens. The currently available HTLV-I assays exhibit inherent difficulties. Since HTLV-I retroviruses bud from cell membranes, there will be human cellular membrane determinants present in the viral envelope. The cross-reactivity of endogenous autoimmune antibody with these determinants leads to a substantial number of false positives. Falsely negative values are also common because the immunoreactive viral envelope protein is often destroyed during the purification of the virus prior to its use in the screening assay. The same problems are inherent in the currently available HIV-1 screening assays.

Expression of recombinant HTLV-I and HIV-1 gene products in bacterial and yeast systems has now been reported and peptides corresponding to specific genes have been isolated. These recombinantly produced peptide antigens are cross-reactive with human sera from patients who are known to be seropositive for HTLV-I and HIV-1. Separate antibody screening assays which utilize recombinantly expressed protein antigens are currently being developed for both HTLV-I and HIV-1.

## SUMMARY OF THE INVENTION

The present invention provides hybrid genes comprising a first DNA sequence coding for a polypeptide containing at least one epitope of the HIV-1 envelope protein which is recognized by antibodies to HIV-1, and a second DNA sequence coding for a polypeptide containing at least one epitope of the HTLV-I envelope protein which is recognized by antibodies to HTLV-I. This hybrid gene is useful for the production of the corresponding hybrid protein, a source of DNA for other purposes, or other related purposes.

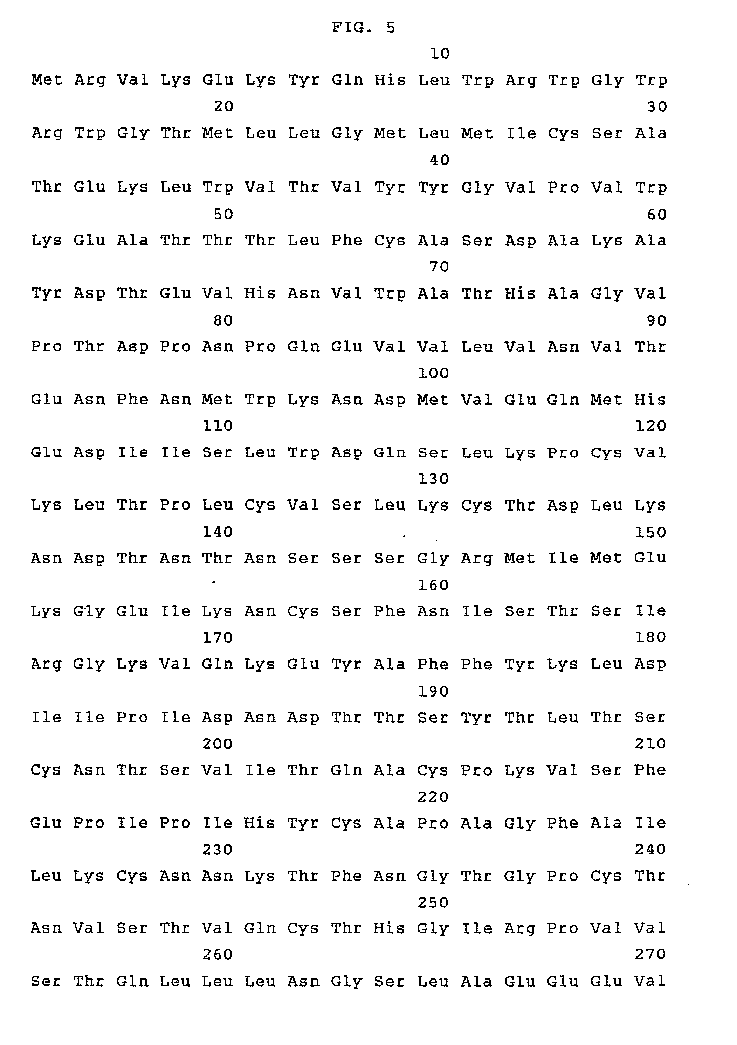

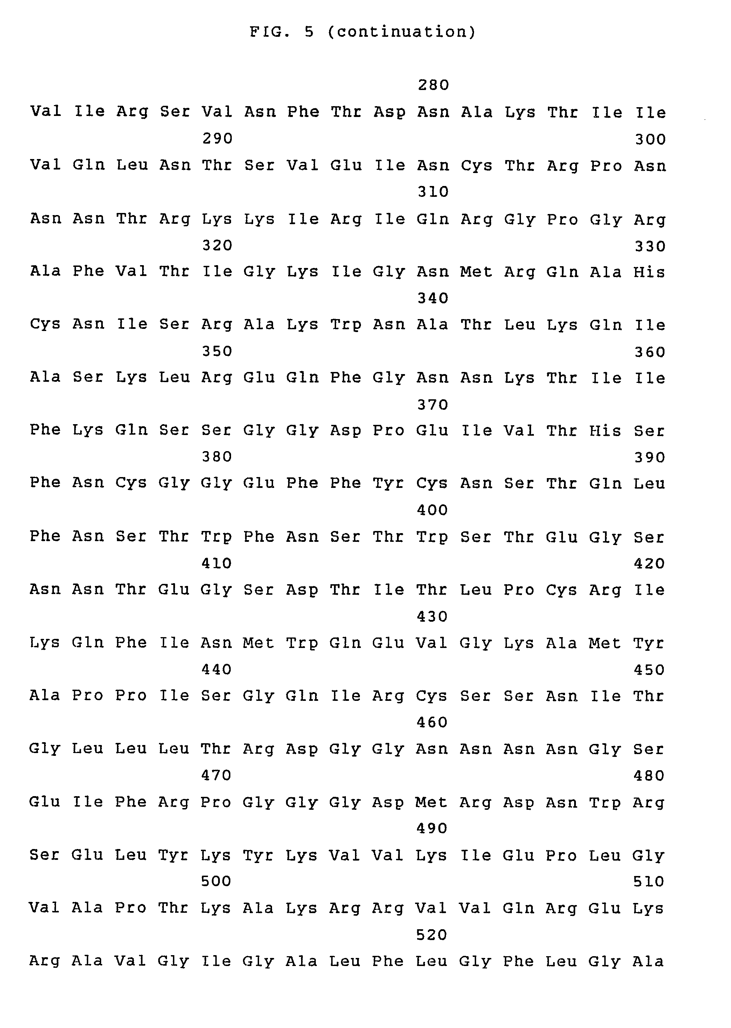

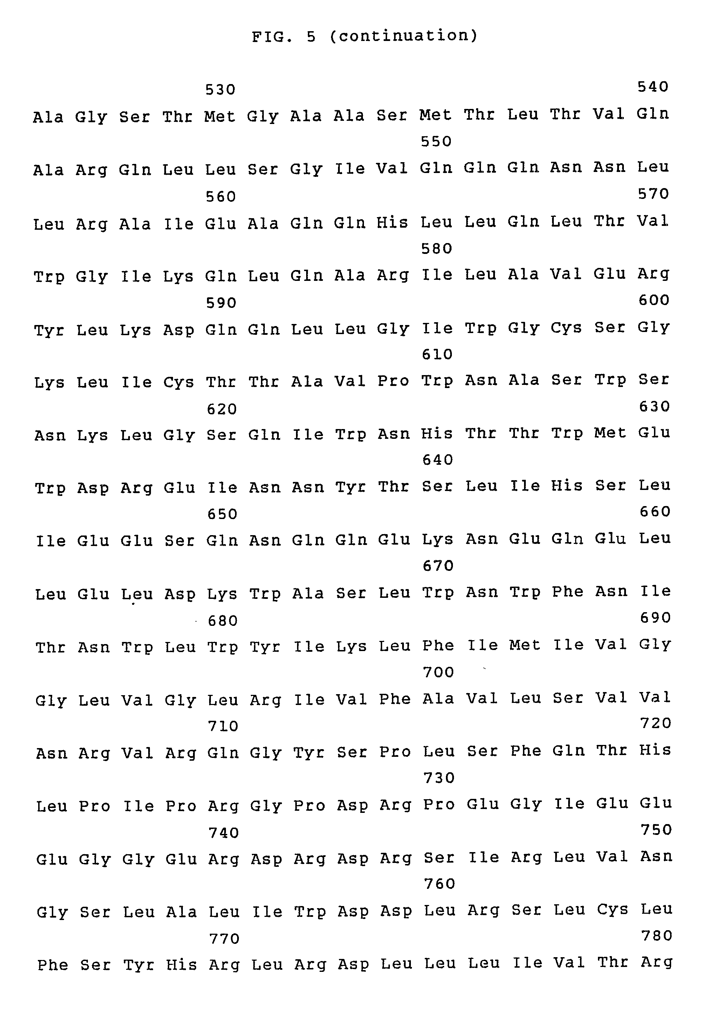

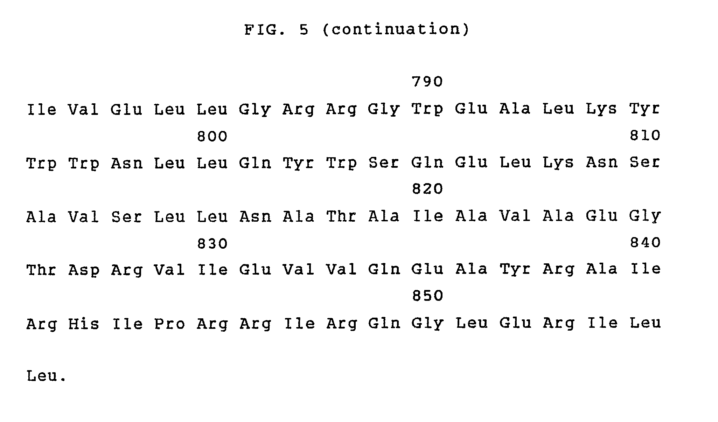

There are two preferred embodiments of the genes. First is a gene in which the first DNA sequence codes for a polypeptide containing the immunodominant region of the HIV-1 gp41 transmembrane protein, preferably approximately amino acids 560-620 of the HIV-1 envelope protein (numbered according to Figure 5), and the second DNA sequence codes for a polypeptide containing approximately amino acids 306-440 of the HTLV-I envelope protein.

The second preferred embodiment is a gene in which the first DNA sequence codes for a polypeptide containing the immunodominant region of the HIV-1 gp41 transmembrane protein, preferably approximately amino acids 560-620 of the HIV-1 envelope protein and the second DNA sequence codes for a polypeptide containing approximately amino acids 201-308 of the HTLV-I envelope protein.

The present invention also provides the corresponding hybrid proteins comprising a first amino acid subsequence containing at least one epitope of the HIV-1 envelope protein which is recognized by antibodies to HIV-1, and a second amino acid subsequence containing at least one epitope of the HTLV-I envelope protein which is recognized by antibodies to HTLV-I. The hybrid proteins of the present invention are useful as antigens to detect the presence of antibodies to the HTLV-I and HIV-1 envelope proteins, as immunogens, as well as other therapeutic or diagnostic purposes.

There are two preferred embodiments of the polypeptides. First is a polypeptide where the first amino acid subsequence contains the immunodominant region of the HIV-1 gp41 transmembrane protein, preferably approximately amino acids 560-620 of the HIV-1 envelope protein (said amino acids numbered in accordance with Figure 5) and the second amino acid subsequence contains approximately amino acids 306-440 of the HTLV-I envelope protein.

Second is a polypeptide where the first amino acid subsequence contains the immunodominant region of the HIV-1 gp41 transmembrane protein, preferably approximately amino acids 560-620 of the HIV-1 envelope protein and the second amino acid subsequence contains approximately amino acids 201-308 of the HTLV-I envelope protein.

The present invention also comprises recombinant vectors comprising the hybrid genes of the present invention, particularly a recombinant vector which is a plasmid.capable of replication in a unicellular host.

The present invention also comprises unicellular hosts carrying the recombinant vectors of the present invention, as well as a method for producing the hybrid proteins of the present invention by culturing a unicellular host containing a recombinant vector of the present invention under appropriate conditions of growth to produce the corresponding hybrid proteins.

The present invention also provides a method for detecting antibodies to HTLV-I envelope proteins, HTLV-II envelope proteins and/or HIV-1 envelope proteins in mammalian body fluids comprising contacting a test sample containing antibodies with a hybrid protein containing at least one epitope of the HIV-1 envelope protein and at least one epitope of the HTLV-I envelope protein and allowing protein-antibody complexes to form and detecting the complexes, thereby demonstrating the presence of antibodies in the sample. Preferred is where the method is an immunoassay such as Western Blotting, Double Antigen Sandwich Method, Radioimmunoassay or, particularly, Enzyme Immunoassay.

The present invention also comprises a diagnostic test kit useful for the detection of antibodies to HIV-1, and/or HTLV-I, or HTLV-II in mammalian body fluids.

Both HTLV-I and HIV-1 are very important human pathogens so a single screening test which measures antibodies to both HTLV-1 and HIV-1 is highly desirable.


## DESCRIPTION OF DRAWINGS


Fig. 1 Illustrates the construction of plasmids HIV-1 pENV(60)-HTLV-I-ENV-1 and -ENV-2 pENV(60)-HTLV-I-ENV1 and-ENV-2. The HTLV-I genome and restriction enzyme fragments of the envelope gene used for subcloning are shown. (For details see Examples 1 and 2). The cloning and expression of HIV-1 pENV(80) has been described by Certa et al., EMBO J. 5; 3051-3056 (1986).

Fig. 2 The HIV-1-HTLV-I translation product of plasmid HIV-1 pENV(60)-HTLV-I-ENV-1 is set forth immediately prior to its complete DNA and amino acid sequence (single letter code designations for amino acids indicate residues which are vector-related and not specific to the envelope genes).

Fig. 3 The HIV-1-HTLV-I translation product of plasmid HIV-1 pENV(60)-HTLV-I-ENV-2 is set forth immediately prior to its complete DNA and amino acid sequence (single letter code designations for amino acids indicate residues which are vector-related and are not specific to the envelope genes).

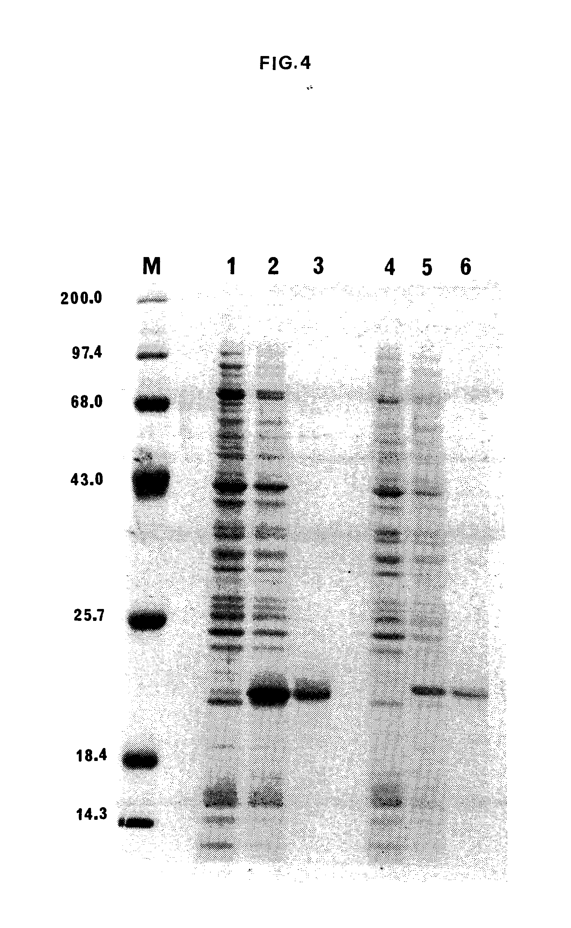

Fig. 4 Illustrates the expression of HIV-1 pENV(60)-HTLV-I-ENV-1 and -ENV-2 fusion proteins and resolution of induced products on SDS-PAGE. Samples were prepared as described in Example 5 and visualized in gels with Coomassie blue staining. The contents of the lanes shown are as follows: E. coli W 3110 cells containing pDMI,1 and HIV-1 pENV(60)-HTLV-I-ENV-1 uninduced (lane 1), 4 hour induced (lane 2) and Sephacryl S-200 purified (lane 3), E. coli Je 5506 cells containing pDMI,1 and HIV-1 pENV(60)-HTLV-I-ENV-2 uninduced (lane 4), 4 hour induced (lane 5), and Sephacryl S-200 purified (lane 6). M represents molecular weight standards.

Fig. 5 Illustrates the amino acid sequence of the HIV-1 envelope protein.

## DETAILED DESCRIPTION

Preferred is a hybrid gene having the DNA sequence:

```
initiation
sequence
ATG AGA GGA TCC GAA GCT CAA CAG CAT CTG CTG CAA CTC ACT GTT
TGG GGT ATC AAA CAG CTC CAG GCT CGA ATT CTG GCT GTT GAA CGT
TAC CTG AAA GAT CAA CAG CTC CTG GGT ATC TGG GGC TGC AGT GGT
AAA CTC ATC TGC ACT ACT GCT GTT CCT TGG AAT GCT TCT TGG TCT
AAT AAG CTT GGA TCC CGC TCC CGC CGA GCG GTA CCG GTG GCG GTC
TGG CTT GTC TCC GCC CTG GCC ATG GGA GCC GGA GTG GCT GGC GGG
ATT ACC GGC TCC ATG TCC CTC GCC TCA GGA AAG AGC CTC CTA CAT
GAG GTG GAC AAA GAT ATT TCC CAG TTA ACT CAA GCA ATA GTC AAA
AAC CAC AAA AAT CTA CTC AAA ATT GCG CAG TAT GCT GCC CAG AAC
AGA CGA GGC CTT GAT CTC CTG TTC TGG GAG CAA GGA GGA TTA TGC
AAA GCA TTA CAA GAA CAG TGC CGT TTT CCG AAT ATT ACC AAT TCC
CAT GTC CCA ATA CTA CAA GAA AGA CCC CCC CTT GAG AAT CGA GTC
CTG ACT GGC TGG GGC CTT AAC TGG GAC CTT GGC CTC TCA CAG TGG
GCT CGA CCT GCA GCC AAG CTC AAG CTT GGC GAG ATT TTC AGG AGC TAA
                              termination sequence
```

Also preferred is a hybrid gene having the DNA sequence:

initiation

sequence

ATG AGA GGA TCC GAA GCT CAA CAG CAT CTG CTG CAA CTC ACT GTT TGG GGT ATC AAA CAG CTC CAG GCT CGA ATT CTG GCT GTT GAA CGT TAC CTG AAA GAT CAA CAG CTC CTG GGT ATC TGG GGC TGC AGT GGT AAA CTC ATC TGC ACT ACT GCT GTT CCT TGG AAT GCT TCT TGG TCT AAT AAG CTT GGA TCC GTC GAG CCC TCT ATA CCA TGG AAA TCA AAA CTC CTG ACC CTT GTC CAG TTA ACC CTA CAA AGC ACT AAT TAT ACT TGC ATT GTC TGT ATC GAT CGT GCC AGC CTC TCC ACT TGG CAC GTC CTA TAC TCT CCC AAC GTC TCT GTT CCA TCC TCT TCT TCT ACC CCC CTC CTT TAC CCA TCG TTA GCG CTT CCA GCC CCC CAC CTG ACG TTA CCA TTT AAC TGG ACC CAC TGC TTT GAC CCC CAG ATT CAA GCT ATA GTC TCC TCC CCC TGT CAT AAC TCC CTC ATC CTG CCC CCC TTT TCC TTG TCA CCT GTT CCC ACC CTA GGA TCC AAG CTT GGC GAG ATT TTC

AGG AGC TAA.                                          termination sequence


As shown above, the hybrid genes of the present invention may additionally contain "initiation" and "termination" DNA sequences. These sequences arise as a result of the recombinant vector and are essential for expression of the hybrid genes in the unicellular host. The initiation sequences are operatively linked to the hybrid genes. Various initiation sequences, all of which may exhibit different nucleotide sequences, may be used with the present hybrid genes and the invention is intended to include the hybrid genes in combination with all these sequences. The preferred initiation DNA sequence of the present invention has the nucleotide sequence:

ATG AGA GGA TCC

The "termination" DNA sequences which may additionally be present after the terminal codon of the hybrid genes also result from the recombinant vectors used for expression of the corresponding gene products. The termination DNA sequences will vary with the recombinant vectors used. The present invention is intended to include all the various termination DNA sequences.

In the hybrid genes of the present invention the two DNA sequences may be fused directly to one another or they may be attached to one another via "linker" sequences. A linker sequence may be inserted between the HIV-1 envelope gene sequence and the HTLV-I envelope gene sequence to maintain the proper reading frame during translation of the hybrid proteins. The present invention is intended to include the various linker DNA sequences which may be used for this purpose.

The preferred hybrid genes of the present invention code for the polypeptides having the amino acid sequences of the formulae:

```
Met Arg Gly Ser Glu Ala Gln Gln His Leu Leu Gln Leu Thr Val
Trp Gly Ile Lys Gln Leu Gln Ala Arg Ile Leu Ala Val Glu Arg
Tyr Leu Lys Asp Gln Gln Leu Leu Gly Ile Trp Gly Cys Ser Gly
Lys Leu Ile Cys Thr Thr Ala Val Pro Trp Asn Ala Ser Trp Ser
Asn Lys Leu Gly Ser Arg Ser Arg Arg Ala Val Pro Val Ala Val
Trp Leu Val Ser Ala Leu Ala MET Gly Ala Gly Val Ala Gly Gly
Ile Thr Gly Ser MET Ser Leu Ala Ser Gly Lys Ser Leu Leu His
Glu Val Asp Lys Asp Ile Ser Gln Leu Thr Gln Ala Ile Val Lys
Asn His Lys Asn Leu Leu Lys Ile Ala Gln Tyr Ala Ala Gln Asn
Arg Arg Gly Leu Asp Leu Leu Phe Trp Glu Gln Gly Gly Leu Cys
Lys Ala Leu Gln Glu Gln Cys Arg Phe Pro Asn Ile Thr Asn Ser
His Val Pro Ile Leu Gln Glu Arg Pro Pro Leu Glu Asn Arg Val
Leu Thr Gly Trp Gly Leu Asn Trp Asp Leu Gly Leu Ser Gln Trp
Ala Arg Pro Ala Ala Lys Leu Lys Leu Gly Glu Ile Phe Arg Ser.
                              terminal amino acids
```

and

```
Met Arg Gly Ser Glu Ala Gln Gln His Leu Leu Gln Leu Thr Val
Trp Gly Ile Lys Gln Leu Gln Ala Arg Ile Leu Ala Val Glu Arg
Tyr Leu Lys Asp Gln Gln Leu Leu Gly Ile Trp Gly Cys Ser Gly
Lys Leu Ile Cys Thr Thr Ala Val Pro Trp Asn Ala Ser Trp Ser
Asn Lys Leu Gly Ser Val Glu Pro Ser Ile Pro Trp Lys Ser Lys
Leu Leu Thr Leu Val Gln Leu Thr Leu Gln Ser Thr Asn Tyr Thr
Cys Ile Val Cys Ile Asp Arg Ala Ser Leu Ser Thr Trp His Val
Leu Tyr Ser Pro Asn Val Ser Val Pro Ser Ser Ser Ser Thr Pro
Leu Leu Tyr Pro Ser Leu Ala Leu Pro Ala Pro His Leu Thr Leu
Pro Phe Asn Trp Thr His Cys Phe Asp Pro Gln Ile Gln Ala Ile
Val Ser Ser Pro Cys His Asn Ser Leu Ile Leu Pro Pro Phe Ser
Leu Ser Pro Val Pro Thr Leu Gly Ser Lys Leu Gly Glu Ile Phe
Arg Ser.                      terminal amino acids
```

These polypeptides may additionally contain sequences of several amino acids which are coded for by the corresponding "termination" or "linker" DNA sequences mentioned previously. The present invention is intended to include the various amino acid sequences coded for by the "termination" or "linker" sequences, none of which affect the functionality of the hybrid polypeptides but occur as a result of expression in the transformed host.

The hybrid proteins themselves may also contain amino acid substitutions if such substitutions do not generally alter the biological activity of the hybrid proteins. Amino acid substitutions in polypeptides which do not essentially alter their biological activities are know in the art and described, e.g. by H. Neurath and R.L. Hill in "The Proteins", Academic Press, New York (1979). The most frequently observed amino acid substitutions are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, Asp/Gly, and vice versa.

Further, given the similarity of the HTLV-I and HTLV-II viruses, antibodies to HTLV-II will also recognize the present hybrid polypeptides. The present invention is also intended to include the detection of antibodies to HTLV-II in mammalian body fluids according to the invention. [see European Patent Application, Publication No. 181,107].

## The hybrid genes and their preparation

The hybrid genes of the present invention are made by fusing a DNA sequence containing at least one epitope of the HTLV-I envelope protein which recognizes antibodies to HTLV-I to a DNA sequence containing at least one epitope of the HIV-1 envelope protein which recognizes antibodies to HIV-1.

The HTLV-I and HIV-1 envelope gene fragments can be obtained from a variety of sources.

The HTLV-I envelope gene codes for a glycoprotein of 61,000 daltons (gp61) which is cleaved into a 46,000 M.W. exterior glycoprotein (gp46) and the 21,000 M.W. transmembrane protein (gp21).

HTLV-I envelope gene fragments which can be used for the manufacture of the hybrid genes are available from a number of sources. For example, in the present invention a plasmid designated pH2Ex was used as a source of HTLV-I envelope gene DNA. This plasmid was originally subcloned from a plasmid designated λCR1 which contains Env, pX, and LTR of HTLV-I as described in Manzari et al., PNAS 80, 1574-1578 (1983) and European Patent Application, Publication No. 181 107.

Plasmid pKS300, which codes for part of the C-terminal region of gp46 of the HTLV-I envelope proteins, and plasmid pKS400, which codes for part of the N-terminal region of gp21 of the HTLV-1 envelope protein can also be used and may be easily derived from the above mentioned plasmid pH2Ex by the skilled artisan utilizing known methods. These plasmids are the subject of European Patent Application, Publication No. 181 107.

Other possible sources of HTLV-I envelope gene fragments include chemical synthesis of the gene sequences according to methods known in the art [Certa et al., EMBO J, 5, 3051-3056 (1986)].

HTLV-I envelope gene fragments can also be obtained from human T-cell lines which have been virally transformed by HTLV-I virions as set forth in Yoshida et al., PNAS 79, 2031-2035 (1982) and Poiesz et al., PNAS 77, 7415-7419 (1980). The DNA fragments can then be isolated by methods known in the art, a cDNA library constructed, and the desired envelope gene fragments can be obtained by probing the cDNA library.

The HIV-1 envelope gene codes for a glycosylated protein (gp) with a molecular weight of 160,000 (gp160) that is processed to gp120 and gp41. HIV-1 envelope gene fragments which can be used to make the fusion gene are widely available. The HIV-1 envelope gene sequence desired can be chemically synthesized according to known methods, or the DNA sequences can be isolated by preparing DNA which is complementary to the mRNA produced from the virally transformed cell lines. The HIV-1 virus has been successfully cultured using the H-9 cell line [PCT application, Publication No. WO 85/04897] and this can be one source of HIV-1 envelope DNA fragments. The entire genome of HIV-1 has been molecularly cloned as set forth in Schüpbach et al., Science 224,503-505 (1984). The complete nucleotide sequence of the genome has been determined as set forth in Ratner et al., Nature 313,277-284 (1985). The particular HIV-1 envelope gene fragment used to construct the hybrid protein of the instant invention was obtained as described in Certa et al., EMBO J. 5,3051-3056 (1986).

To construct the hybrid genes of the present invention it is necessary to excise the desired portions of the HTLV-I envelope gene and the HIV-1 envelope gene utilizing restriction endonucleases which will "cut" the DNA sequences at specific sites. Examples 1 and 2 illustrate the means by which the hybrid genes are constructed utilizing this methodology.

Synthetic hybrid genes can also be manufactured. Oligonucleotide fragments are synthesized which, when assembled correctly, form a hybrid gene coding for the hybrid protein. The fragments can be hybridized and ligated in pre-determined stages to construct the synthetic gene. The synthetic gene can be provided with suitable restriction enzyme sites and cloned into vectors specifically designed to maximize expression of the synthetic gene.

In order to construct said hybrid genes which code for the desired hybrid proteins several criteria should be observed. Firstly, trinucleotide codons should be used which are acceptable to or preferably used in the cells, in particular E. coli. Secondly, it would be desirable to have restriction enzyme recognition sites at the termini of the molecule so as to allow insertion into a vector of plasmid or phage origin. Moreover, restriction sites should be selected which allow the use of well-understood cloning and expression vectors, such as plasmids of the pDS-family. Thirdly, a series of restriction endonuclease recognition sites should be introduces to exchange and modify parts of the gene very easily and to allow the expression or certain parts of it. Fourthly, the synthesis should not be unnecessarily complicated and illegitimate cross-hybridizations should be minimized in order to facilitate gene assembly.

The hybrid genes of the present invention can be introduced in any convenient expression vector of plasmid or phage origin in a manner known per se. Convenient expression vectors of plasmid or phage origin are mentioned e.g., in the laboratory manual "Molecular Cloning" by Maniatis et al., Cold Spring Harbor Laboratory, 1982.

## The recombinant vectors and transformed hosts

Once the hybrid genes have been constructed they can be introduced into any convenient expression vector, plasmid or phage in any manner known per se. Convenient recombinant vectors of plasmid or phage origin are mentioned, for example, in the Laboratory Manual "Molecular Cloning", Maniatis et al., Cold Spring Harbor Laboratory, 1982.

E. coli strains containing plasmids useful for these constructions are, e.g. E.coli M15 containing plasmids pDS5/RBSII,3A + 5A and pDMI,1 (DSM 3517), E. coli M15 containing plasmids pDS6/RBSII,3A + 5A and pDMI,1 (DSM 3518) or E. coli TB1 containing plasmid pA-ENV-20 (DSM 3516). These strains are all freely available upon request from Deutsche Sammlung von Mikroorganismen (DSM) in Göttingen.

As discussed previously, the hybrid genes, when inserted into the appropriate site of the recombinant vectors, may be fused to DNA sequences which are not part of the structural genes themselves. It is only required, however, that the inserted gene sequences, in the transformed hosts, produce hybrid proteins or analogs obtained by amino acid substitutions.

Methods for expressing DNA sequences which code for the hybrid proteins of the present invention are well known, e.g. from Maniatis et al, supra. They include transforming an appropriate host with a recombinant vector having the said DNA sequence operatively linked to an expression control DNA sequence, culturing the transformed host under appropriate conditions of growth and extracting and isolating the desired hybrid protein from the culture. The skilled artisan may select from these known methods those which are most effective for a particular gene expression without departing from the scope of the present invention.

The selection of a particular host for use in this invention is dependent upon a number of factors recognized by the art. These include, for example, compatibility with the chosen expression vector, toxicity of the proteins encoded for the hybrid plasmid, ease of recovery of the desired protein, expression characteristics, biosafety and costs. Within these general guidelines, examples of useful bacterial hosts are gram-negative and gram-positive bacteria, especially strains of E. coli. The most preferred host cells of the present invention are E. coli M15 (described as DZ291 in M.R. Villarejo et al., J. Bacteriol. 120, 466-474 [1974]) and E. coli Je 5506 (described in Hirota et al., PNAS 74, 1417-1420 [1977]). However, other E. coli strains, such as E. coli 294 (ATCC No. 31446), E. coli RR1 (ATCC No. 31343) and E. coli W3110 (ATCC No. 27325), can also be used.

As produced in E. coli, the hybrid proteins of the present invention are largely confined to insoluble aggregates of the bacterium, a fact which greatly facilitates purification of these proteins. To isolate the hybrid proteins of the present invention the bacterial cells are disrupted or lysed and the insoluble proteins are recovered by centrifugation. Substantial purification can then be obtained by sequential washing of the precipitate with buffer, detergent and salt solutions followed by the use of standard protein purification techniques.

For small amounts of material such as samples taken for polyacrylamide gel electrophoretic analysis, the cells can be disrupted by treatment with a detergent such as sodium dodecyl sulfate (SDS). Larger quantities of the proteins can be recovered by sonication, or by other mechanically disruptive means, such as the French pressure cell. Preferably, the cells are lysed by chemical and enzymatic means, such as EDTA or EGTA and lysozyme.

Preferably the insoluble protein precipitate is washed in three separate steps although it is foreseeable that certain of these steps could be combined, rearranged or eliminated. The first step of washing the precipitate may be done with a buffer solution, preferably 10mM Tris, pH 8.0. In the second step the precipitate is washed with a detergent solution, preferably 1.0% Triton X-100. The third and final wash is with a salt solution, preferably 7M Guanidine-HCl.

The hybrid protein precipitate resulting from the final wash solution can then be further purified by conventional protein purification techniques including but not limited to gel filtration, chromatography, preparative flat-bed iso-electric focusing, gel-electrophoresis, high performance liquid chromatography (hereinafter HPLC: including ion exchange, gel filtration and reverse phase chromatography), and affinity chromatography, e.g. on dye bound carrier or on Sepharose coupled with monoclonal antibodies against said hybrid protein. Preferably the hybrid protein precipitate is further purified by size exclusion chromatography using Superose 12 or Sephacryl S-200 gels.

The methods of this invention entail a number of steps which, in logical sequence, include (1) preparation of the genes encoding the hybrid gene sequences, (2) insertion of these genes into appropriate cloning vehicles to produce recombinant vectors containing said hybrid gene sequences, (3) transfer of the recombinant cloning vehicles into compatible host organisms, (4) selection and growth of such modified hosts that can express the inserted gene sequences, (5) identification and purification of the gene products, (6) use of the gene products to detect antibodies against HIV-1 or HTLV-I or related viruses.

The hybrid proteins of the present invention can be produced by conventionally known methods. The processes by which the novel hybrid proteins can be produced can be divided into three groups: (1) chemical synthesis, preferably solid-phase synthesis; (2) preparation of the fusion genes prepared by chemical synthesis which are inserted into a host and expression of the hybrid proteins by the host; and (3) the corresponding hybrid genes are obtained from sources such as plasmids or clones as set forth above, and inserted into a host and the proteins are expressed by the host.

The hybrid proteins of the present invention can be synthesized by utilizing the general solid phase methods of Merrifield (Journal of American Chemical Society 85, 2149 [1963]).

The hybrid proteins can also be produced by recombinant methods by inserting the hybrid gene sequences into an appropriate recombinant vector, and transforming a host. The transformant is then grown under appropriate culture conditions to yield the corresponding hybrid proteins. Example 3, infra, illustrates the expression of the recombinant proteins of the present invention, which is generally described by Maniatis et al., supra.

Methods for detecting antibodies to HIV-1, HTLV-I or HTLV-II in mammalian subjects

The hybrid proteins of the present invention can be used to detect the presence of antibodies to both HIV-1 envelope protein and HTLV-I or related viruses (such as HTLV-II) envelope proteins in mammalian body fluids such as, serum, tears, semen, vaginal secretions and saliva. The invention encompasses a diagnostic method for testing mammalian body fluids for the presence of antibodies to HIV-1, HTLV-I, or related viruses and a test kit useful in this method.

One method in which antibodies against the HIV-1, HTLV-I or related viruses (such as HTLV-II) can be detected is the so-called "Western Blotting" analysis (Tobin et al., PNAS 76, 4350-4354 (1979)]. According to this technique a hybrid protein of the present invention is transferred electrophoretically from the SDS-polyacrylamide gel onto nitrocellulose paper. The nitrocellulose paper is then treated with the test sample. After washing, the nitrocellulose paper is treated with an anti-human IgG labeled with peroxidase. The peroxidase is then determined by a suitable substrate, e.g. with o-phenylenediamine. Radioactive or fluorescent labels may also be used.

A more convenient technique for the determination of antibodies against the HIV-1 or HTLV-I or HTLV-II virus using a hybrid protein of the present invention is an enzyme-linked immunosorbant assay (ELISA). Such assay comprises:

(a) immobilizing a hybrid polypeptide of the present invention on a solid support;

(b) contacting a test sample with the immobilized polypeptide of step (a) and allowing immobilizing protein-antibody complex to form;

(c) washing away unbound material from the complexes of step (b); and

(d) detecting such complexes by the addition of a labeled reagent capable of selectively detecting human antibodies, such as labeled Staphylococcus aureus protein A or anti-human IgG, thereby demonstrating the presence of antibodies to HIV-1 envelope protein or HTLV-1 envelope protein in the sample.

Suitable solid supports are the inner wall of test vessels (test tube, titer plates or cuvettes of glass or artificial material) as well as the surface of solid bodies (rods of glass and artificial material, rods with terminal thickening, rods with terminal lobes or lamellae). Beads of glass or artificial material are especially suitable solid phase carriers.

Useful labels are any detectable functionalities which do not interfere with the binding of reagent and its binding partner. Numerous labels are known for use in ELISA assays and other immuno assays such as horseradish peroxidase, radioisotopes such as $^{14}C$ and $^{125}I$, fluorophores such as rare earth chelates for fluorescein, spin labels and the like.

In a preferred embodiment of the present invention an ELISA (EIA) assay is run in a test kit as described below utilizing the hybrid proteins of the present invention immobilized on beads. To the coated spheres, patient samples, and positive and negative controls are added and allowed to incubate at 37°C for 30 minutes. The beads are washed to remove unbound antibodies and then anti-human IgG coupled to horseradish peroxidase (HRP) are added. The HRP conjugate is allowed to react for 15 minutes at 37°C and then the spheres are washed to remove unbound HRP conjugate. HRP substrate, o-phenylenediamine (OPD), is added to the spheres and allowed to react for 15 minutes at room temperature. The enzyme reaction is terminated by addition of $H_2SO_4$ and the resulting yellow-brown color produced by the HRP is read in a spectrophotometer. The optical density at 492 nm indicates the presence of HRP-conjugate bound to the human IgG which is in turn bound to the antigen on the plate.

Antibodies to HIV-1 envelope protein or HTLV-I envelope protein can also be determined utilizing the hybrid proteins of the present invention with an enzyme immunological test according to the so-called "Double-Antigen-Sandwich-Method". This method is based on the work of Maiolini, R.I., as described in Immunological Methods 20, 25-34 (1978). According to this method, the test sample is contacted with a solid support upon which the hybrid proteins of the present invention, labeled with peroxidase, are coated. The immunological reaction can then be performed in one or in two steps. If the immunological reaction is performed in two steps then a washing step is performed between the two incubations. After the immunological reaction or reactions a washing step is performed. Thereafter the peroxidase is determined with a substrate, e.g. with o-phenylene diamine.

Any positive result which may be obtained by utilizing the method of the present invention to detect antibodies to HIV-1 envelope or HTLV-I envelope protein in mammalian body fluids, can be confirmed by separately testing the sample for antibodies to HIV-1 envelope or HTLV-I envelope.

The present invention will be further described i.n connection with the following examples which are set forth for the purposes of illustration only.

Example 1

Construction of plasmid HIV-1 pENV(60)-HTLV-I-ENV-1

Plasmid pH2Ex was used as a source of HTLV-I envelope gene DNA. This plasmid was originally subcloned from λCR1 which contains Env, pX and LTR of HTLV-I (Manzari et al., supra). Plasmid pBSENV was constructed by inserting a 715 bp XhoI restriction fragment into the unique saII site of expression vector pDS56/RBSII (European Patent Application, Publication No. 282 042)]. The inserted DNA fragment encodes the amino acids 201-440 of the HTLV-1 envelope gene.

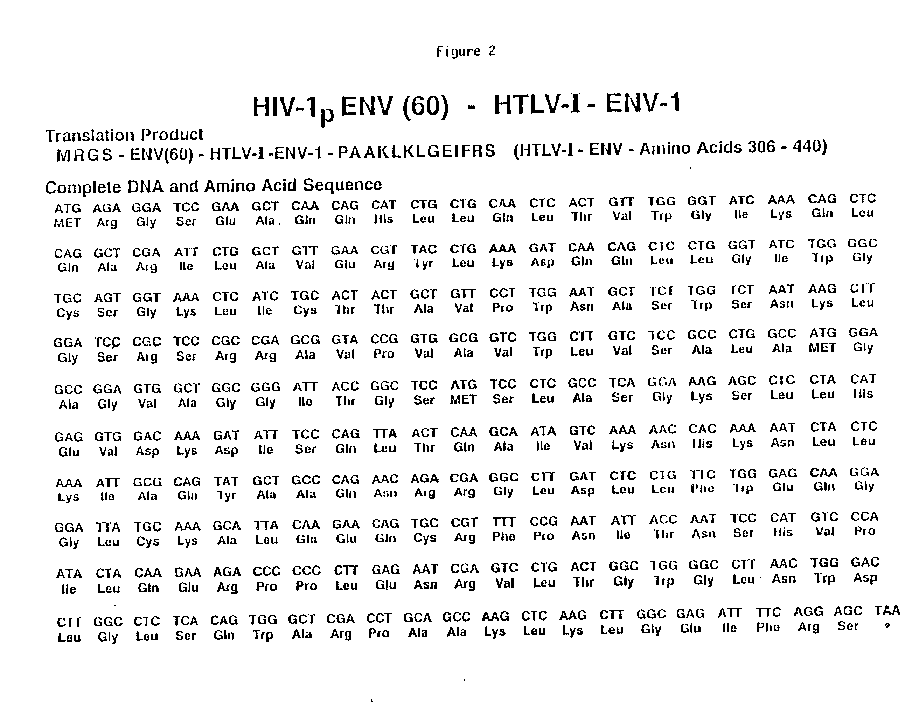

Plasmid HIV-1 pENV(60)-HTLV I-ENV-1 was constructed by removing the 413 bp BamHI/HindIII restriction fragment from pBSENV, filling in the recessed 3'-OH ends with the Klenow fragment of DNA Polymerase I, ligating synthetic HindIII linkers of appropriate size to maintain the reading frame (New England Biolabs), and inserting this HindIII fragment into expression vector pDS5/RBSII ENV(60). This expression vector was derived from clone pDS5/RBSII ENV(80) by removing the HindIII restriction fragment and recircularizing the remaining vector. This vector now encodes the N-terminal 60 amino acids of a chemically synthesized DNA fragment which represents an immunodominant region of the HIV 1 gp41 transmembrane protein. Plasmid HIV-1 pENV( 60)-HTLV-I-ENV-1 encodes for a hybrid protein with a predicted molecular weight of 23.2 Kd containing 60 amino acids of the HIV-1 envelope gene (homologous to amino acids 560-620) and 134 amino acids of the HTLV-I envelope gene (amino acids 306-440). Additional four amino acids at the N-terminus are contributed by vector sequences as well as additional thirteen amino acids which are expressed on the C-terminus of the fusion protein before a termination codon is recognized (see Fig. 2).

Example 2

Construction of plasmid HIV-1 pENV(60)-HTLV-I-ENV-2.

10

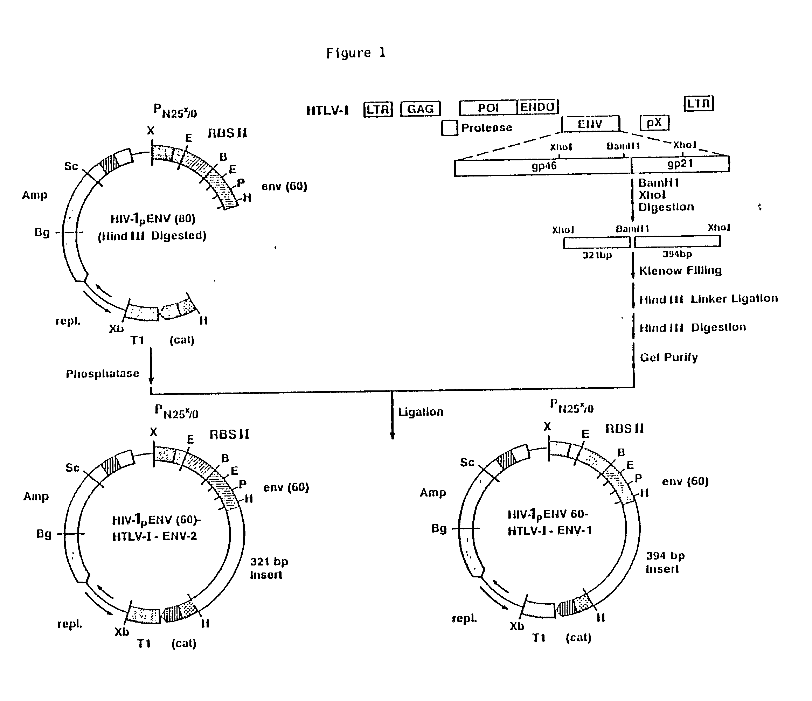

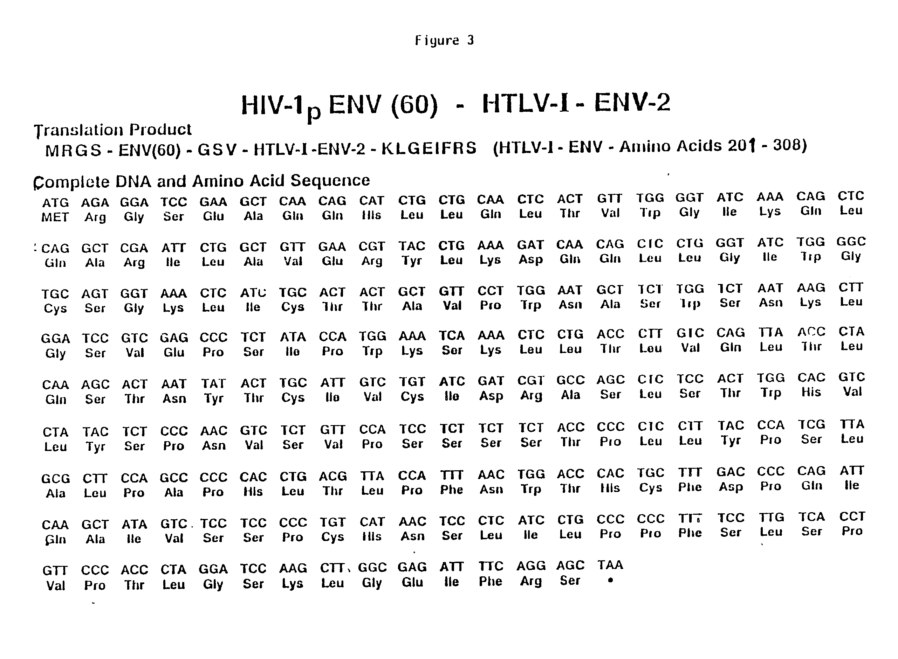

Plasmid HIV-1 pENV(60)-HTLV-I-ENV-2 codes for the C-terminus of gp46, the exterior glycoprotein of HTLV-I (amino acids 201-308). It was constructed by removing a 328 bp BamHI restriction fragment from pBSENV, attaching HindIII linkers which maintain the reading frame of the pDS expression vector used and inserting the resulting fragment into the HindIII site of pDS5/RBSII ENV(60) (Fig. 1 and 3).

## Example 3

### Expression of Recombinant Proteins.

Expression of the inserted genes was induced by the addition of isopropyl-$\beta$-D-thiogalactopyranoside (IPTG) to actively growing bacterial cultures as described by Certa et al., supra, with some modifications. Cultures were grown to an OD600 of 0.6-0.7 at 37°C, induced by addition of IPTG to 0.5 mM, and aliquots removed after 2-4 hours incubation. After collecting cells by centrifugation, equivalent amounts of whole cell lysate are run on 12% SDS-PAGE gels and protein bands visualized by staining with Coomassie Brilliant Blue (R-250) (Figure 4). Whole cell lysate samples were prepared by resuspending 1.5 ml of centrifuged culture fluid in 2x Laemmli sample buffer and boiled.

## Example 4

### Purification of Recombinant Proteins.

Purification of the recombinant fusion proteins was achieved through a modification of the procedure of Manne at al. [PNAS 82, .376 (1985)]. Briefly, IPTG induced cell pellets were resuspended in PBS/5 mM EDTA/25% sucrose/1% Triton X-100/1 mM DTT, pH 7.5, at 2 ml per gram of cell paste. Lysozyme was added to 1 mg/ml and the suspension frozen and thawed 3 times. DNase 1 was added to 0.4 mg/ml, incubated on ice for 20 min., and the lysate was centrifuged at 25,000 x g for 15 min. at 4°C. The resulting pellet was washed 4 times with 25% sucrose/1% Triton X-100/1mM DTT (20 ml). The pellet was then washed with PBS/1.75M Guanidine HCl/5mM DTT, pH 7.5, and the pellet solubilized in 3 ml 10mM Tris/5mM DTT/7M Guanidine HCL, pH 8.0. Insoluble materials were removed by centrifugation. The supernatant was diluted 1:15 with 10 mM Tris/5 mM DTT, incubated for 10 min. at room temp. and centrifuged at 12,000 x g for 10 min. at 4°C. The pellet was washed once with 20 ml 10 mM Tris/5 mM DTT, pH 8.0, and slowly dissolved in 125 mM Tris/5 mM DTT/4% SDS/0.02% NaN3, pH 6.8, at room temperature overnight. In certain cases, further purification was accomplished by chromatography on a Sephacryl S-200 (Pharmacia) column equilibrated in 25 mM Tris/1 mM EDTA/100 mM NaCl/0.1% SDS/0.02% NaN3, pH 8.0. Fractions were analyzed by SDS-PAGE and those containing the protein of interest were pooled.

## Example 5

### Testing of Clinical Samples

Sera tested were obtained from normal blood donors and AIDS and ARC patients who were previously shown to be seropositive. HTLV-I seropositive samples were obtained from Japan. Pooled positive control sera for HIV-1 and HTLV-I was obtained from Western States Plasma.

## Procedure for Bead Coating.

### Reagents:

1. 0.05 M Carbonate - Bicarbonate buffer, pH 9.5.
2. Polystyrene beads (alcohol washed).
3. 1% Tween 20 in 0.05 carbonate-bicarbonate buffer, pH 9.5.
4. HIV-1 HTLV-I fusion protein in 25 mM Tris, 1 mM EDTA, 100 mM NaCl, 0.1% SDS, 0.02% NaN$_3$, pH 8.0.
5. Coating Solution: HIV-1 HTLV-I protein is diluted with 0.05 M carbonate-bicarbonate buffer, pH 9.5 to a final concentration of 10 $\mu$g/ml.

### Protocol:

1. Add 500 beads, alcohol washed (70 gr) to 100 ml of 1% Tween 20 in 0.05 carbonate/bicarbonate buffer, pH 9.5
2. Incubate at 37°C overnight (18 hrs.). Aspirate solution and wash 5 times with 200 ml of deionized water.
Remove last wash by suction.
3. Cover wet beads with 100 ml of coating solutions containing 10 $\mu$g/ml HIV-1 HTLV-I fusion protein.
4. Incubate for 20 hrs. at 37°C.
5. Remove coating solution by suction.
6. Wash 5 times with 200 ml of deionized water.
7. Remove last wash by suction.
8. Dry beads in tumbler at 37°C for 3 hrs. and store at 2.8°C.

## Combination Antibody Screen.

### Reagents:

1. HIV-1 HTLV-I-ENV protein coated beads: Polystyrene frosted bead, coated with 10 $\mu$g/ml HIV-1 pENV(60)-HTLV-I-ENV-1 fusion protein solution.
2. Sample diluent: 20% newborn calf serum, 0.5% Tween 20, 0.01% goat IgG, 0.01% Thimerosal, 5 mM EDTA in 0.02 M phosphate buffered saline, pH 7.0.
3. HIV-1 low positive control: heat inactivated human serum positive for antibody to HIV, 0.1% azide, 0.01% Thimerosal.
4. HIV-1 high positive control: heat inactivated human serum positive for antibody to HIV, 0.1% azide, 0.01% Thimerosal.
5. HIV-1/HTLV-I negative control: heat inactivated human serum negative for antibody to HIV-1 and HTLV-I, 0.1% azide, 0.01% Thimerosal.
6. HTLV-I positive control: heat inactivated human serum positive for antibody to HTLV-I, 0.1% azide, 0.01% Thimerosal.
7. Conjugate reagent: goat anti-human IgG peroxidase (horseradish) labelled, diluted 1:900 (dilution may vary with titer) in 0.1 M Tris acetate, containing 20% treated fetal calf serum, 0.04% 4-amino antipyrine, 1% Tween 20 (polysorbate 20), 0.1% Kathon (v/v), pH 7.3.
8. Substrate buffer: 0.1 M potassium citrate, .02% hydrogen peroxide, 0.01% Kathon, pH 5.25.
9. OPD tablets: 10 mg o-phenylenediamine/tablet.
10. Stopping reagent: 1N sulfuric acid in deionized water.

### Assay Procedure:

1. Dispense 10 µl of specimen plus 400 ul of sample diluent (1:41 dilution) into the reaction tube.

2. Add 1 bead.

3. Incubate using Resa COBAS shaker/incubator for 30 min. at 37° C.

4. Wash tubes in washer using deionized water.

5. Add 250 µl of conjugate reagent.

6. Incubate using Resa COBAS shaker/incubator for 15 min. at 37° C.

7. Approximately 10 minutes before the end of immunological incubation, prepare the OPD substrate solution by dissolving OPD tablets in substrate buffer (add 1 tablet per 5 ml of substrate buffer).

8. Wash tubes in the washer using deionized water.

9. Add 250 µl already combined OPD/substrate.

10. Shake rack.

11. Incubate 15 min. at room temperature in the dark.

12. Add 1 ml of stopping reagent per tube.

13. Read tubes at 492 nm in Resa COBAS spectrophotometer.

The assay results are shown in Tables 1 and 2.

TABLE 1

| IMMUNOREACTIVITY OF RECOMBINANTLY EXPRESSED HTLV-I AND HIV-1 ENVELOPE PROTEINS (O.D. = 492) | | | | | |
|---|---|---|---|---|---|
| SAMPLES | NO. | HIV-1 pENV(80) | HIV-1 pENV(60)-HTLV-I-ENV-1 | HIV-1 pENV(60)-HTLV-I-ENV-2 | HIV-1 pENV(60)-HTLV-I-ENV-1 + ENV-2 |
| HIV-1 POSITIVES | 1 | 1.134 | 1.304 | 0.959 | 2.459 |
| | 2 | 4.471 | 3.157 | 3.137 | 4.918 |
| | 3 | 4.224 | 3.211 | 2.957 | 4.582 |
| | 4 | 3.981 | 4.650 | 2.998 | 4.600 |
| | 5 | 4.422 | 3.395 | 3.263 | 4.858 |
| HTLV-I POSITIVES | 1 | 0.102 | 1.983 | 0.690 | 3.772 |
| | 2 | 0.120 | 1.287 | 1.350 | 3.135 |
| | 3 | 0.074 | 0.685 | 1.071 | 2.757 |
| | 4 | 0.075 | 1.860 | 0.700 | 3.608 |
| | 5 | 0.143 | 2.604 | 0.300 | 4.577 |
| NEGATIVES | 1 | 0.177 | 0.051 | 0.133 | 0.166 |
| | 2 | 0.290 | 0.061 | 0.175 | 0.218 |
| | 3 | 0.178 | 0.062 | 0.120 | 0.204 |
| | 4 | 0.195 | 0.082 | 0.165 | 0.172 |
| | 5 | 0.146 | 0.057 | 0.092 | 0.137 |

EP 0 345 792 A2

TABLE 2

| DETECTION OF HUMAN ANTIBODY AGAINST HIV-1 AND HTLV-I USING RECOMBINANTLY EXPRESSED ENVELOPE FUSION PROTEINS | | | | | | |
|---|---|---|---|---|---|---|
| SAMPLES | HIV-1 ASSAY | | | HIV-1/HTLV-I COMBINATION ASSAY | | |
| | MEAN (O.D. 492) | RANGE | REACTIVE RATIO | MEAN | RANGE | REACTIVE |
| RATIO | | | | | | |
| *HTLV-I POSITIVES (n = 22) | 0.132 | 0.074-0.188 | 0/22 | 2.5 | 0.315-4.608 | 22/22 |
| HIV-1 POSITIVES (n = 100) | 3.980 | 1.134-5.206 | 100/100 | 4.576 | 2.586-5.691 | 100/100 |
| NEGATIVES (n = 100) | 0.195 | 0.096-0.396 | 0/100 | 0.194 | 0.053-0.338 | 0/100 |

*TEN SAMPLES WERE TESTED AT 1:100 DILUTION (INSTEAD OF 1:40) DUE TO INSUFFICIENT SAMPLE VOLUME.

While the invention has been described in connection with the preferred embodiment, it is not intended to limit the scope of the invention to the particular form set forth, but, on the contrary, it is intended to cover such alternatives, modifications, and equivalents as may be included within the spirit and scope of the invention as defined by the appended claims.

**Claims**

1. A hybrid gene comprising a first DNA sequence coding for a polypeptide containing at least one epitope of the HIV-1 envelope protein which is recognized by antibodies to HIV-1 and a second DNA sequence coding for a polypeptide containing at least one epitope of the HTLV-I envelope protein which is recognized by antibodies to HTLV-I.

2. The gene of claim 1 wherein the first DNA sequence codes for a polypeptide containing the immunodominant region of the HIV-1 gp41 transmembrane protein.

3 The gene of claim 2 wherein the DNA sequence codes for approximately amino acids 560-620 of the HIV-1 envelope protein.

4. The gene of claim 3 which contains a initiation DNA sequence operatively linked to the hybrid gene.

5. The gene of claim 4 wherein the initiation DNA sequence is:
ATG AGA GGA TCC.

6. The gene of claim 5 wherein the second DNA sequence codes for a polypeptide containing approximately amino acids 306-440 of the HTLV-1 envelope protein, said gene having the nucleotide sequence
ATG AGA GGA TCC GAA GCT CAA CAG CAT CTG CTG CAA CTC ACT GTT TGG GGT ATC AAA CAG CTC CAG GCT CGA ATT CTG GCT GTT GAA CGT TAC CTG AAA GAT CAA CAG CTC CTG GGT ATC TGG GGC TGC AGT GGT AAA CTC ATC TGC ACT ACT GCT GTT CCT TGG AAT GCT TCT TGG TCT AAT AAG CTT GGA TCC CGC TCC CGC CGA GCG GTA CCG GTG GCG GTC TGG CTT GTC TCC GCC CTG GCC ATG GGA GCC GGA GTG GCT GGC GGG ATT ACC GGC TCC ATG TCC CTC GCC TCA GGA AAG AGC CTC CTA CAT GAG GTG GAC AAA GAT ATT TCC CAG TTA ACT CAA GCA ATA GTC AAA AAC CAC AAA AAT CTA CTC AAA ATT GCG CAG TAT GCT GCC CAG AAC AGA CGA GGC CTT GAT CTC CTG TTC TGG GAG CAA GGA GGA TTA TGC AAA GCA TTA CAA GAA CAG TGC CGT TTT CCG AAT ATT ACC AAT TCC CAT GTC CCA ATA CTA CAA GAA AGA CCC CCC CTT GAG AAT CGA GTC CTG ACT GGC TGG GGC CTT AAC TGG GAC CTT GGC CTC TCA CAG TGG GCT CGA CCT GCA GCC AAG CTC AAG CTT GGC GAG ATT TTC AGG AGC TAA

7. The gene of claim 5, wherein the second DNA sequence codes for a polypeptide containing approximately amino acids 201-308 of the HTLV-I envelope protein, said gene having the nucleotide sequence
ATG AGA GGA TCC GAA GCT CAA CAG CAT CTG CTG CAA CTC ACT GTT TGG GGT ATC AAA CAG CTC CAG GCT CGA ATT CTG GCT GTT GAA CGT TAC CTG AAA GAT CAA CAG CTC CTG GGT ATC TGG GGC TGC AGT GGT AAA CTC ATC TGC ACT ACT GCT GTT CCT TGG AAT GCT TCT TGG TCT AAT AAG CTT GGA TCC GTC GAG CCC TCT ATA CCA TGG AAA TCA AAA CTC CTG ACC CTT GTC CAG TTA ACC CTA CAA AGC ACT AAT TAT ACT TGC ATT GTC TGT ATC GAT CGT GCC AGC CTC TCC ACT TGG CAC GTC CTA TAC TCT CCC AAC GTC TCT GTT CCA TCC TCT TCT TCT ACC CCC CTC CTT TAC CCA TCG TTA GCG CTT CCA GCC CCC CAC CTG ACG TTA CCA TTT AAC TGG ACC CAC TGC TTT GAC CCC CAG ATT CAA GCT ATA GTC TCC TCC CCC TGT CAT AAC TCC CTC ATC CTG CCC CCC TTT TCC TTG TCA CCT GTT CCC ACC CTA GGA TCC AAG CTT GGC GAG ATT TTC AGG AGC TAA.

8. A hybrid polypeptide comprising a first amino acid subsequence containing at least one epitope of the HIV-1 envelope protein which is recognized by antibodies to HIV-1, and a second amino acid subsequence containing at least one epitope of the HTLV-I envelope protein which is recognized by antibodies to HTLV-I.

9. The polypeptide of claim 8, wherein the first subsequence contains the immunodominant region of the HIV-1 gp41 transmembrane protein.

10. The polypeptide of claim 9, wherein the first subsequence contains approximately amino acids 560-620 of the HIV-1 envelope protein.

11. The polypeptide of claim 10 wherein the second amino acid subsequence contains approximately amino acids 201-308 of the HTLV-I envelope protein, said polypeptide having the amino acid sequence Met Arg Gly Ser Glu Ala Gln Gln His Leu Leu Gln Leu Thr Val Trp Gly ILe Lys Gln Leu Gln Ala Arg Ile Leu Ala Val Glu Arg Tyr Leu Lys Asp Gln Gln Leu Leu Gly Ile Trp Gly Cys Ser Gly Lys Leu Ile Cys Thr Thr Ala Val Pro Trp Asn Ala Ser Trp Ser Asn Lys Leu Gly Ser Val Glu Pro Ser Ile Pro Trp Lys Ser Lys Leu Leu Thr Leu Val Gln Leu Thr Leu Gln Ser Thr Asn Tyr Thr Cys Ile Val Cys Ile Asp Arg Ala Ser Leu Ser Thr Trp His Val Leu Tyr Ser Pro Asn Val Ser Val Pro Ser Ser Ser Ser Thr Pro Leu Leu Tyr Pro Ser Leu Ala Leu Pro Ala Pro His Leu Thr Leu Pro Phe Asn Trp Thr His Cys Phe Asp Pro Gln Ile Gln Ala Ile Val Ser Ser Pro Cys His Asn Ser Leu Ile Leu Pro Pro Phe Ser Leu Ser Pro Val Pro Thr Leu Gly Ser Lys Leu Gly Glu Ile Phe Arg Ser.

12. The polypeptide of claim 10, wherein the second amino acid subsequence contains approximately amino acids 306-440 of the HTLV-I envelope protein, said polypeptide having the amino acid sequence
Met Arg Gly Ser Glu Ala Gln Gln His Leu Leu Gln Leu Thr Val Trp Gly Ile Lys Gln Leu Gln Ala Arg Ile Leu Ala Val Glu Arg Tyr Leu Lys Asp Gln Gln Leu Leu Gly Ile Trp Gly Cys Ser Gly Lys Leu Ile Cys Thr Thr Ala Val Pro Trp Asn Ala Ser Trp Ser Asn Lys Leu Gly Ser Arg Ser Arg Arg Ala Val Pro Val Ala Val Trp Leu Val Ser Ala Leu Ala MET Gly Ala Gly Val Ala Gly Gly Ile Thr Gly Ser MET Ser Leu Ala Ser Gly Lys Ser Leu Leu His Glu Val Asp Lys Asp Ile Ser Gln Leu Thr Gln Ala Ile Val Lys Asn His Lys Asn Leu Leu Lys Ile Ala Gln Tyr Ala Ala Gln Asn Arg Arg Gly Leu Asp Leu Leu Phe Trp Glu Gln Gly Gly Leu Cys Lys Ala Leu Gln Glu Gln Cys Arg Phe Pro Asn Ile Thr Asn Ser His Val Pro Ile Leu Gln Glu Arg Pro Pro Leu Glu Asn Arg Val Leu Thr Gly Trp Gly Leu Asn Trp Asp Leu Gly Leu Ser Gln Trp Ala Arg Pro Ala Ala Lys Leu Lys Leu Gly Glu Ile Phe Arg Ser.

13. A recombinant vector comprising a hybrid gene which codes for a hybrid polypeptide containing at least one epitope of the HIV-1 envelope protein which is recognized by antibodies to HIV-1 and at least one epitope of the HTLV-I envelope protein which is recognized by antibodies to HTLV-I.

14. The recombinant vector of claim 13 which is a plasmid capable of replication in a unicellular host.

15. The recombinant vector of claim 14 which is capable of replication in an E. coli strain.

16. The recombinant vector of claim 15 in which the vector is a member of the plasmid pENV family.

17. The recombinant vector of claim 16 which is plasmid HIV-1 pENV(60)-HTLV-I-ENV-1.

18. The recombinant vector of claim 16 which is plasmid HIV-1 pENV(60)-HTLV-I-ENV-2.

19. A unicellular host containing a recombinant vector comprising a hybrid gene coding for a hybrid polypeptide containing at least one epitope of the HIV-1 envelope protein which is recognized by antibodies to HIV-1, and at least one epitope of the HTLV-I envelope protein which is recognized by antibodies to HTLV-I.

20. The host of claim 19 which is an E. coli strain.

21. The E. coli strain of claim 20 which is E. coli M15.

22. A method for producing a hybrid polypeptide containing at least one epitope of the HIV-1 envelope protein, and at least one epitope of the HTLV-I envelope protein comprising culturing a unicellular host containing a recombinant vector which contains a gene coding for said hybrid polypeptide under appropriate conditions of growth so that said polypeptide is expressed and isolating said polypeptide.

23. A method for detecting antibodies to HTLV-I or HTLV-II envelope protein or HIV-1 envelope protein in mammalian body fluids comprising contacting a test sample with a hybrid polypeptide containing at least one epitope of the HIV-1 envelope protein and at least one epitope of the HTLV-1 envelope protein and allowing protein-antibody complexes to form and detecting the complexes.

24. The method of claim 23 wherein the complexes are determined in an immunoassay.

25. The method of claim 24 which is an immunoassay selected from the group consisting of Western Blotting, Double Antigen Sandwich Assay, Radioimmunoassay, or Enzyme Immunoassay.

26. The method of claim 25 comprising:

a) immobilizing a hybrid polypeptide of claims 8 to 12 on a solid support;

b) contacting a mammalian body fluid sample with the immobilized polypeptide of step (a) and allowing the protein-antibody complex to form;

c) washing away unbound material from the complexes of step (b); and

d) detecting such complexes by the addition of labeled reagent capable of selectively detecting human antibodies.

27. The method of claim 26 wherein the solid support is a bead.

28. The method of claim 27 wherein the reagent capable of selectively detecting human antibodies is labeled anti-human IgG.

29. The method of claim 28 wherein the label is a radiolabel.

30. The method of claim 29 wherein the label is an enzyme label.

31. The method of claim 30 wherein any positive test result is confirmed by testing the sample separately for antibodies to HTLV-I or HIV-1 envelope protein

32. The use of a hybrid protein of claims 8 to 12 for testing mammalien body fluids for the presence of antibodies to HTLV-I, HTLV-II. or HIV-1 envelope proteins.

33. A diagnostic kit useful for measuring antibodies to HTLV-1, HTLV-II, or HIV-1 envelope protein in mammalian body fluids comprising:

a) a container with a hybrid protein of claims 8 to 12 immobilized on a solid support;

b) a container with labeled reagent capable of selectively detecting human antibodies;

c) containers with positive and negative controls; and

d) a container with sample diluent.

34. The test kit of claim 33 wherein the solid support is a bead.

Figure 1

Figure 2

# HIV-1$_p$ ENV (60) - HTLV-I - ENV-1

**Translation Product**

MRGS - ENV(60) - HTLV-I -ENV-1 - PAAKLKLGEIFRS   (HTLV-I - ENV - Amino Acids 306 - 440)

**Complete DNA and Amino Acid Sequence**

| | | | | | | | | | | | | | | | | | | | |
|---|---|---|---|---|---|---|---|---|---|---|---|---|---|---|---|---|---|---|---|
| ATG | AGA | GGA | TCC | GAA | GCT | CAA | CAG | CAT | CTG | CTG | CAA | CTC | ACT | GTT | TGG | GGT | ATC | AAA | CAG | CTC |
| MET | Arg | Gly | Ser | Glu | Ala. | Gln | Gln | His | Leu | Leu | Gln | Leu | Thr | Val | Trp | Gly | Ile | Lys | Gln | Leu |

| | | | | | | | | | | | | | | | | | | | |
|---|---|---|---|---|---|---|---|---|---|---|---|---|---|---|---|---|---|---|---|
| CAG | GCT | CGA | ATT | CTG | GCT | GTT | GAA | CGT | TAC | CTG | AAA | GAT | CAA | CAG | CTC | CTG | GGT | ATC | TGG | GGC |
| Gln | Ala | Arg | Ile | Leu | Ala | Val | Glu | Arg | Tyr | Leu | Lys | Asp | Gln | Gln | Leu | Leu | Gly | Ile | Trp | Gly |

| | | | | | | | | | | | | | | | | | | | |
|---|---|---|---|---|---|---|---|---|---|---|---|---|---|---|---|---|---|---|---|
| TGC | AGT | GGT | AAA | CTC | ATC | TGC | ACT | ACT | GCT | GTT | CCT | TGG | AAT | GCT | TCT | TGG | TCT | AAT | AAG | CTT |
| Cys | Ser | Gly | Lys | Leu | Ile | Cys | Thr | Thr | Ala | Val | Pro | Trp | Asn | Ala | Ser | Trp | Ser | Asn | Lys | Leu |

| | | | | | | | | | | | | | | | | | | | |
|---|---|---|---|---|---|---|---|---|---|---|---|---|---|---|---|---|---|---|---|
| GGA | TCC | CGC | TCC | CGC | CGA | GCG | GTA | CCG | GTG | GCG | GTC | TGG | CTT | GTC | TCC | GCC | CTG | GCC | ATG | GGA |
| Gly | Ser | Arg | Ser | Arg | Arg | Ala | Val | Pro | Val | Ala | Val | Trp | Leu | Val | Ser | Ala | Leu | Ala | MET | Gly |

| | | | | | | | | | | | | | | | | | | | |
|---|---|---|---|---|---|---|---|---|---|---|---|---|---|---|---|---|---|---|---|
| GCC | GGA | GTG | GCT | GGC | GGG | ATT | ACC | GGC | TCC | ATG | TCC | CTC | GCC | TCA | GGA | AAG | AGC | CTC | CTA | CAT |
| Ala | Gly | Val | Ala | Gly | Gly | Ile | Thr | Gly | Ser | MET | Ser | Leu | Ala | Ser | Gly | Lys | Ser | Leu | Leu | His |

| | | | | | | | | | | | | | | | | | | | |
|---|---|---|---|---|---|---|---|---|---|---|---|---|---|---|---|---|---|---|---|
| GAG | GTG | GAC | AAA | GAT | ATT | TCC | CAG | TTA | ACT | CAA | GCA | ATA | GTC | AAA | AAC | CAC | AAA | AAT | CTA | CTC |
| Glu | Val | Asp | Lys | Asp | Ile | Ser | Gln | Leu | Thr | Gln | Ala | Ile | Val | Lys | Asn | His | Lys | Asn | Leu | Leu |

| | | | | | | | | | | | | | | | | | | | |
|---|---|---|---|---|---|---|---|---|---|---|---|---|---|---|---|---|---|---|---|
| AAA | ATT | GCG | CAG | TAT | GCT | GCC | CAG | AAC | AGA | CGA | GGC | CTT | GAT | CTC | CTG | TTC | TGG | GAG | CAA | GGA |
| Lys | Ile | Ala | Gln | Tyr | Ala | Ala | Gln | Asn | Arg | Arg | Gly | Leu | Asp | Leu | Leu | Phe | Trp | Glu | Gln | Gly |

| | | | | | | | | | | | | | | | | | | | |
|---|---|---|---|---|---|---|---|---|---|---|---|---|---|---|---|---|---|---|---|
| GGA | TTA | TGC | AAA | GCA | TTA | CAA | GAA | CAG | TGC | CGT | TTT | CCG | AAT | ATT | ACC | AAT | TCC | CAT | GTC | CCA |
| Gly | Leu | Cys | Lys | Ala | Leu | Gln | Glu | Gln | Cys | Arg | Phe | Pro | Asn | Ile | Thr | Asn | Ser | His | Val | Pro |

| | | | | | | | | | | | | | | | | | | | |
|---|---|---|---|---|---|---|---|---|---|---|---|---|---|---|---|---|---|---|---|
| ATA | CTA | CAA | GAA | AGA | CCC | CCC | CTT | GAG | AAT | CGA | GTC | CTG | ACT | GGC | TGG | GGC | CTT | AAC | TGG | GAC |
| Ile | Leu | Gln | Glu | Arg | Pro | Pro | Leu | Glu | Asn | Arg | Val | Leu | Thr | Gly | Trp | Gly | Leu | Asn | Trp | Asp |

| | | | | | | | | | | | | | | | | | | | | |
|---|---|---|---|---|---|---|---|---|---|---|---|---|---|---|---|---|---|---|---|---|
| CTT | GGC | CTC | TCA | CAG | TGG | GCT | CGA | CCT | GCA | GCC | AAG | CTC | AAG | CTT | GGC | GAG | ATT | TTC | AGG | AGC | TAA |
| Leu | Gly | Leu | Ser | Gln | Trp | Ala | Arg | Pro | Ala | Ala | Lys | Leu | Lys | Leu | Gly | Glu | Ile | Phe | Arg | Ser | • |

EP 0 345 792 A2

Figure 3

# HIV-1$_p$ ENV (60)  -  HTLV-I - ENV-2

**Translation Product**

MRGS - ENV(60) - GSV - HTLV-I-ENV-2 - KLGEIFRS   (HTLV-I - ENV - Amino Acids 201 - 308)

## Complete DNA and Amino Acid Sequence

```
ATG AGA GGA TCC GAA GCT CAA CAG CAT CTG CTG CAA CTC ACT GTT TGG GGT ATC AAA CAG CTC
MET Arg Gly Ser Glu Ala Gln Gln His Leu Leu Gln Leu Thr Val Trp Gly Ile Lys Gln Leu

CAG GCT CGA ATT CTG GCT GTT GAA CGT TAC CTG AAA GAT CAA CAG CTC CTG GGT ATC TGG GGC
Gln Ala Arg Ile Leu Ala Val Glu Arg Tyr Leu Lys Asp Gln Gln Leu Leu Gly Ile Trp Gly

TGC AGT GGT AAA CTC ATC TGC ACT ACT GCT GTT CCT TGG AAT GCT TCT TGG TCT AAT AAG CTT
Cys Ser Gly Lys Leu Ile Cys Thr Thr Ala Val Pro Trp Asn Ala Ser Trp Ser Asn Lys Leu

GGA TCC GTC GAG CCC TCT ATA CCA TGG AAA TCA AAA CTC CTG ACC CTT GTC CAG TTA ACC CTA
Gly Ser Val Glu Pro Ser Ile Pro Trp Lys Ser Lys Leu Leu Thr Leu Val Gln Leu Thr Leu

CAA AGC ACT AAT TAT ACT TGC ATT GTC TGT ATC GAT CGT GCC AGC CTC TCC ACT TGG CAC GTC
Gln Ser Thr Asn Tyr Thr Cys Ile Val Cys Ile Asp Arg Ala Ser Leu Ser Thr Trp His Val

CTA TAC TCT CCC AAC GTC TCT GTT CCA TCC TCT TCT TCT ACC CCC CTC CTT TAC CCA TCG TTA
Leu Tyr Ser Pro Asn Val Ser Val Pro Ser Ser Ser Ser Thr Pro Leu Leu Tyr Pro Ser Leu

GCG CTT CCA GCC CCC CAC CTG ACG TTA CCA TTT AAC TGG ACC CAC TGC TTT GAC CCC CAG ATT
Ala Leu Pro Ala Pro His Leu Thr Leu Pro Phe Asn Trp Thr His Cys Phe Asp Pro Gln Ile

CAA GCT ATA GTC TCC TCC CCC TGT CAT AAC TCC CTC ATC CTG CCC CCC TTT TCC TTG TCA CCT
Gln Ala Ile Val Ser Ser Pro Cys His Asn Ser Leu Ile Leu Pro Pro Phe Ser Leu Ser Pro

GTT CCC ACC CTA GGA TCC AAG CTT GGC GAG ATT TTC AGG AGC TAA
Val Pro Thr Leu Gly Ser Lys Leu Gly Glu Ile Phe Arg Ser •
```

EP 0 345 792 A2

FIG.4

## FIG. 5

```
                                   10
Met Arg Val Lys Glu Lys Tyr Gln His Leu Trp Arg Trp Gly Trp
            20                                          30
Arg Trp Gly Thr Met Leu Leu Gly Met Leu Met Ile Cys Ser Ala
                                   40
Thr Glu Lys Leu Trp Val Thr Val Tyr Tyr Gly Val Pro Val Trp
            50                                          60
Lys Glu Ala Thr Thr Thr Leu Phe Cys Ala Ser Asp Ala Lys Ala
                                   70
Tyr Asp Thr Glu Val His Asn Val Trp Ala Thr His Ala Gly Val
            80                                          90
Pro Thr Asp Pro Asn Pro Gln Glu Val Val Leu Val Asn Val Thr
                                   100
Glu Asn Phe Asn Met Trp Lys Asn Asp Met Val Glu Gln Met His
            110                                         120
Glu Asp Ile Ile Ser Leu Trp Asp Gln Ser Leu Lys Pro Cys Val
                                   130
Lys Leu Thr Pro Leu Cys Val Ser Leu Lys Cys Thr Asp Leu Lys
            140                                         150
Asn Asp Thr Asn Thr Asn Ser Ser Ser Gly Arg Met Ile Met Glu
                                   160
Lys Gly Glu Ile Lys Asn Cys Ser Phe Asn Ile Ser Thr Ser Ile
            170                                         180
Arg Gly Lys Val Gln Lys Glu Tyr Ala Phe Phe Tyr Lys Leu Asp
                                   190
Ile Ile Pro Ile Asp Asn Asp Thr Thr Ser Tyr Thr Leu Thr Ser
            200                                         210
Cys Asn Thr Ser Val Ile Thr Gln Ala Cys Pro Lys Val Ser Phe
                                   220
Glu Pro Ile Pro Ile His Tyr Cys Ala Pro Ala Gly Phe Ala Ile
            230                                         240
Leu Lys Cys Asn Asn Lys Thr Phe Asn Gly Thr Gly Pro Cys Thr
                                   250
Asn Val Ser Thr Val Gln Cys Thr His Gly Ile Arg Pro Val Val
            260                                         270
Ser Thr Gln Leu Leu Leu Asn Gly Ser Leu Ala Glu Glu Glu Val
```

## FIG. 5 (continuation)

```
                                     280
Val Ile Arg Ser Val Asn Phe Thr Asp Asn Ala Lys Thr Ile Ile
                290                                     300
Val Gln Leu Asn Thr Ser Val Glu Ile Asn Cys Thr Arg Pro Asn
                                     310
Asn Asn Thr Arg Lys Lys Ile Arg Ile Gln Arg Gly Pro Gly Arg
                320                                     330
Ala Phe Val Thr Ile Gly Lys Ile Gly Asn Met Arg Gln Ala His
                                     340
Cys Asn Ile Ser Arg Ala Lys Trp Asn Ala Thr Leu Lys Gln Ile
                350                                     360
Ala Ser Lys Leu Arg Glu Gln Phe Gly Asn Asn Lys Thr Ile Ile
                                     370
Phe Lys Gln Ser Ser Gly Gly Asp Pro Glu Ile Val Thr His Ser
                380                                     390
Phe Asn Cys Gly Gly Glu Phe Phe Tyr Cys Asn Ser Thr Gln Leu
                                     400
Phe Asn Ser Thr Trp Phe Asn Ser Thr Trp Ser Thr Glu Gly Ser
                410                                     420
Asn Asn Thr Glu Gly Ser Asp Thr Ile Thr Leu Pro Cys Arg Ile
                                     430
Lys Gln Phe Ile Asn Met Trp Gln Glu Val Gly Lys Ala Met Tyr
                440                                     450
Ala Pro Pro Ile Ser Gly Gln Ile Arg Cys Ser Ser Asn Ile Thr
                                     460
Gly Leu Leu Leu Thr Arg Asp Gly Gly Asn Asn Asn Asn Gly Ser
                470                                     480
Glu Ile Phe Arg Pro Gly Gly Gly Asp Met Arg Asp Asn Trp Arg
                                     490
Ser Glu Leu Tyr Lys Tyr Lys Val Val Lys Ile Glu Pro Leu Gly
                500                                     510
Val Ala Pro Thr Lys Ala Lys Arg Arg Val Val Gln Arg Glu Lys
                                     520
Arg Ala Val Gly Ile Gly Ala Leu Phe Leu Gly Phe Leu Gly Ala
```

FIG. 5 (continuation)

```
                  530                                        540
Ala Gly Ser Thr Met Gly Ala Ala Ser Met Thr Leu Thr Val Gln
                                      550
Ala Arg Gln Leu Leu Ser Gly Ile Val Gln Gln Gln Asn Asn Leu
                  560                                        570
Leu Arg Ala Ile Glu Ala Gln Gln His Leu Leu Gln Leu Thr Val
                                      580
Trp Gly Ile Lys Gln Leu Gln Ala Arg Ile Leu Ala Val Glu Arg
                  590                                        600
Tyr Leu Lys Asp Gln Gln Leu Leu Gly Ile Trp Gly Cys Ser Gly
                                      610
Lys Leu Ile Cys Thr Thr Ala Val Pro Trp Asn Ala Ser Trp Ser
                  620                                        630
Asn Lys Leu Gly Ser Gln Ile Trp Asn His Thr Thr Trp Met Glu
                                      640
Trp Asp Arg Glu Ile Asn Asn Tyr Thr Ser Leu Ile His Ser Leu
                  650                                        660
Ile Glu Glu Ser Gln Asn Gln Gln Glu Lys Asn Glu Gln Glu Leu
                                      670
Leu Glu Leu Asp Lys Trp Ala Ser Leu Trp Asn Trp Phe Asn Ile
                  680                                        690
Thr Asn Trp Leu Trp Tyr Ile Lys Leu Phe Ile Met Ile Val Gly
                                      700
Gly Leu Val Gly Leu Arg Ile Val Phe Ala Val Leu Ser Val Val
                  710                                        720
Asn Arg Val Arg Gln Gly Tyr Ser Pro Leu Ser Phe Gln Thr His
                                      730
Leu Pro Ile Pro Arg Gly Pro Asp Arg Pro Glu Gly Ile Glu Glu
                  740                                        750
Glu Gly Gly Glu Arg Asp Arg Asp Arg Ser Ile Arg Leu Val Asn
                                      760
Gly Ser Leu Ala Leu Ile Trp Asp Asp Leu Arg Ser Leu Cys Leu
                  770                                        780
Phe Ser Tyr His Arg Leu Arg Asp Leu Leu Leu Ile Val Thr Arg
```

## FIG. 5 (continuation)

                                    790
Ile Val Glu Leu Leu Gly Arg Arg Gly Trp Glu Ala Leu Lys Tyr
            800                                         810
Trp Trp Asn Leu Leu Gln Tyr Trp Ser Gln Glu Leu Lys Asn Ser
                                    820
Ala Val Ser Leu Leu Asn Ala Thr Ala Ile Ala Val Ala Glu Gly
            830                                         840
Thr Asp Arg Val Ile Glu Val Val Gln Glu Ala Tyr Arg Ala Ile
                                    850
Arg His Ile Pro Arg Arg Ile Arg Gln Gly Leu Glu Arg Ile Leu

Leu.